# EUROPEAN PATENT APPLICATION

(11) **EP 1 208 848 A1**
(43) Date of publication of application: **29.05.2002**
(21) Application number: 01309685.4
(22) Date of filing: 16.11.2001
(51) Int. Cl.: A61K 38/47, A61K 31/70

(54) **Stable lactase compositions**

(30) Priority: 17.11.2000 US 715463
(71) Applicant: McNeil-PPC, Inc., Skillman, NJ 08558-9418 (US)
(72) Inventor: Bruce, Richard D., Rydal, PA 19046-3339 (US); Walter, James, Ambler, PA 19002 (US)
(74) Representative: Mercer, Christopher Paul

(57) **Abstract**

The present invention relates to a composition and associated methods thereof for hydrolyzing lactose in a human in need thereof by oral administration having a lactose hydrolyzing amount of β-galactosidase, an excipient, and less than about 10 wt% of a reducing sugar, wherein the composition undergoes a change in yellowness of about 45% or less after seven days at 40°C/75% RH as determined by ASTM D1925.

## Description

### FIELD OF THE INVENTION

The present invention relates to solid lactase compositions having improved stability. More particularly, the present invention relates to more stable lactase compositions for hydrolyzing lactose that contain, among other ingredients, β-galactosidase; an excipient; less than about 10% reducing sugar; and avoids the use of deliquescent excipient combinations.

### BACKGROUND OF THE INVENTION

Lactose, or milk sugar, is a disaccharide carbohydrate that is hydrolyzed during the digestive process to glucose and galactose. The enzyme that catalyses this hydrolysis is lactase, which is also described as β-galactosidase or lactase-phlorizin hydrolase.

Although lactase is normally present in the intestinal juices and mucosa, investigations have shown that a significant portion of the population is lactose intolerant or, in other words, β-galactosidase deficient. As a result, there has been demand for a dietary supplement containing lactose-hydrolyzing enzymes.

Currently marketed lactase supplements, or solid compositions, contain lactase enzyme as an active ingredient and excipients, such as trisodium citrate, dicalcium phosphate, microcrystalline cellulose, mannitol, magnesium stearate, colloidal silicone dioxide, flavor, and sugar, e.g., dextrose and sucrose. Such supplements, however, have been observed to display brown spots, or browning, which is accelerated at greater than ambient temperature and/or elevated humidity. Such browning decreases the shelf life of such compositions.

As a result, non-chemical methods have been employed to extend the shelf life of such compositions. Such methods include, but are not limited to, protective packaging, such as foil pouches, desiccant inserts in the packaging, and temperature and humidity-controlled environments during manufacturing, transport, and pre-consumer sale storage. While these methods generally inhibit the formation of browning when compared to traditional methods, they are relatively costly and do not address browning from a composition standpoint.

What is needed, therefore, is a new formulation for a solid lactase composition that does not display brown spots or browning at greater than ambient temperature and/or elevated humidity. What is also needed is a new composition for a solid lactase composition that does not require special packaging and/or transportation.

It has been surprisingly found that solid lactase compositions having improved stability without the need for special non-chemical shelf life extending methods can be produced from lactase, an excipient, and less than about 10% of a reducing sugar.

### SUMMARY OF THE INVENTION

Briefly, there is provided by the present invention a composition for hydrolyzing lactose in a human in need thereof by oral administration having a lactose hydrolyzing amount of β-galactosidase, an excipient, and less than about 10 wt% of a reducing sugar, wherein the composition undergoes a change in yellowness of about 45% or less after seven days at 40°C/75% RH as determined by ASTM D1925.

### DETAILED DESCRIPTION OF THE INVENTION

Lactose hydrolyzing lactase enzymes are known to be produced by various yeasts, bacteria and fungi. Among the organisms heretofore disclosed as useful for this purpose are yeasts, such as, *Saccharomyces fragilis, Torula cremoris* and *Torula utilis*, bacteria, such as, *Escherichia coli* and *Lactobacillus bulgaricus,* fungi, such as, *Aspergillus oryzae, Aspergillus flavus* and *Aspergillus niger,* and various other microorganisms, such as, those described in U.S. Pat. Nos. 2,681,858, 2,781,266, and 2,809,113. The lactase enzyme preparations produced by these organisms generally have pH optimums on the alkaline side or in the weakly acid pH range of about 5-7. Yeasts, which are the primary source of commercial lactases, are known to produce lactases having pH optimums of about 7. Most of these conventional lactase enzyme preparations contain traces of other proteins and compounds in admixture therewith, which are naturally occurring materials contained in the source matrix and carried through the enzyme isolation process. When lactase is referred to herein, it is such an admixture that is referred to.

As seen, lactase enzymes are commercially produced as biological products that have variability in potency from batch to batch, which requires adjustment with an excipient in order to produce a uniform commercial product. Excipients are typically added to the lactase enzyme product and the amount is adjusted from batch to batch to produce a lactase enzyme product of the target potency. Typically, excipients for lactase enzyme products are selected from any inert pharmaceutical excipient, including, sugars, starches, cellulose and inorganic salts. More particularly, typical excipients include dextrose, mannitol, calcium phosphate, sodium citrate and microcrystalline cellulose.

It is, however, an object of the present invention to decrease, preferably substantially eliminate, the use of reducing sugars as excipients and deliquescent excipient combinations in solid lactase compositions. As described more particularly herein, it has been found that such elimination confers improved stability, cost reduction, and decrease in size of certain forms of lactase compositions, e.g., tablet, capsule, sachet, and the like.

It is an additional object of the present invention to reduce browning of lactase compositions. Such reduction in browning is determined by methods known to those in the art. Preferably, the difference in yellowness index as determined after seven days at 40°C/75% RH by ASTM D1925 compared to yellowness index as determined after seven days at 25°C/60% RH by ASTM D1925 is used as a measurement of the reduction in browning.

Suitable lactase for use herein include, a lactase isolated from *Saccharomyces lactis,* by Gist-Brocade in Delft, Holland, and sold by Enzyme Development Corporation, New York, N.Y.; a lactase from *Aspergillus oryzae*, Lactase Y-400, produced by K. K. Yakult Honsha; a lactase from *Aspergillus oryzae*, Plexazym LA 1, produced by Roehm GmbH; a lactase from *Aspergillus oryzae*, produced by Shinnihon Kagaku Kogyo Co.; a lactase from *Kluyveromyces fragilis*, produced by Sturges Enzymes, Selby, North Yorkshire, England; a lactase from *Aspergillus oryzae*, Takamine lactase, produced by Miles Laboratories, Inc., Elkhart, IN.; a lactase from *Kluyveromyces fragilis* produced by Novo Enzymes, Bagsvaerd, Denmark, and a lactase from *Aspergillus oryzae*, e.g., Lactase F "Amano" 100, produced by Amano Pharmaceutical Co., Ltd. Naka-ku, Nagoya, Japan. These suppliers and others offer, generally, lactase composition, including a diluent, having a potency of between 14,000 and 100,000 FCC lactase units/gram.

"Shelf-life," as used herein for a lactase composition, is the time it takes for the lower 95% confidence interval of the product's chemical and physical characteristics, e.g., color, smoothness, appearance, and potency, plotted versus time to fall below a predetermined lower specification limit. Physical appearance of the tablets is a characteristic that affects consumer acceptance of the product. Thus, it can be seen that shelf-life is a function of the stability of the formula against browning as well as lactase content uniformity in the tablets and the inherent lactase chemical stability. It is an object of the present invention to formulate a lactase containing tablet composition having improved shelf life.

Because the physical appearance of the composition can change, the viability of such a composition is suspect. Thus, it is also an object of the present invention for the lactase composition to exhibit improved physical stability with regard to discoloration resistance, reduced tendency to pick up environmental moisture, and the like.

Accordingly, it is preferred that the solid lactase composition of the present invention has less than about 10% of a reducing sugar. Such reducing sugars include, but are not limited to, dextrose (glucose), galactose, xylose, arabinose, mannose. and fructose. More preferable are compositions having less than about 5% of a reducing sugar. Most preferable are compositions that are substantially free of reducing sugars.

It has been found that avoiding certain combinations of excipients that are surprisingly deliquescent, e.g., trisodium citrate and dextrose in combination, reduces environmental moisture uptake in solid lactase compositions. For purposes of this invention, "excipient" and "excipients" are interchangeable and mean hydrophobic excipients and hydrophilic excipients as exemplified herein.

Hydrophobic excipients, such as, e.g., dicalcium phosphate and talc, were surprisingly found to stablize the composition against color changes at elevated temperature and humidity conditions. Such excipients are believed to provide additional reduction in environmental moisture uptake. Reducing environmental moisture uptake avoids the attending physical property degradation of the composition, such as, e.g., softening, caking, pitting, or stickiness. Suitable hydrophobic excipients include, but are not limited to, talc, calcium phosphate, calcium sulfate, calcium carbonate, magnesium hydroxide, magnesium carbonate, and the like. Typically, the hydrophobic excipients are present in the solid lactase composition of the present invention in an amount of from about 10% to about 75% by weight, preferably from about 45% to about 75% by weight, and more preferably from about 45% to about 65% by weight.

Hydrophilic non-water soluble excipients, such as, e.g., microcrystalline ccllulose and starch, have been surprisingly found to add resistance to discoloration of the present invention at elevated temperature and humidity conditions. It is believed that such excipients act as a wicking agent to sequester environmental moisture ingress to the solid lactase composition of the present invention. Suitable hydrophilic excipients include, but arc not limited to, cellulose derivatives, such as cross-linked carboxymethyl cellulose, starch derivatives, such as sodium starch glycolate, maltodextrins, modified starches and the like. Typically, the hydrophilic non-water soluble excipients are present in the solid lactase composition of the present invention in an amount from about 10 wt% to about 75 wt%, preferably from about 45 wt% to about 75 wt%, and more preferably from about 45 wt% to about 65 wt%.

A preferred hydrophilic non-water soluble excipient, microcrystalline cellulose, is manufactured by the controlled hydrolysis of alpha-cellulose, obtained as a pulp from fibrous plant materials, with dilute mineral acid solutions. Following hydrolysis, the cellulose is purified by filtration and the aqueous slurry is spray dried to form dry, porous particles of a broad size distribution. Suitable microcrystalline cellulose will have an average particle size of from about 20 to about 200 µm. Microcrystalline cellulose is available from several suppliers. Suitable microcrystalline cellulose includes Avicel PH 101, Avicel PH 102, Avicel PH 103, Avicel PH 105 and Avicel PH 200, manufactured by FMC Corporation. Preferably, the microcrystalline cellulose is present in the solid lactase composition of the present invention in an amount of from about 45% to about 75% by weight, more preferably from about 45% to about 65% by weight.

According to the present invention, the improved solid lactase composition may be obtained by mixing lactase, at least one excipient, and less than about 10% of a reducing sugar. Preferably, the composition contains from about 25 wt % to about 35 % by weight lactase and from about 0.3 wt% to about 75 wt% excipient. More preferably, the composition optionally contains at least one of the following excipients: about 7 wt% to about 8 wt % trisodium citrate, 45 wt% to about 65 wt% microcrystalline cellulose, about 10 to about 15 wt% dicalcium phosphate, about 10 wt% to about 15 wt% starch, about 0.5 wt% magnesium stearate, and about 0.3 wt% colloidal silicon dioxide. Subsequently, other desired ingredients may be added to obtain a mixture suitable for compressing.

The list of possible other desired ingredients is extensive, but in the case of compressing, tablets should preferably include a lubricant in the composition or provide some external means for in-press lubrication during compression, such as, e.g., die-pocket injection, wherein the lubricant is injected into the die cavity. This list might include conventional solid fillers or carriers, such as, compressible carbohydrates, which include polyhedric alcohols, such as mannitol, sorbitol, xylitol, maltitol, and the like, non-reducing sugars, and mixtures thereof; binders, such as cellulose, cellulose derivatives, polyvinyl pyrrolidone, polyethylene glycol, gelatin, natural or synthetic gums, such as xanthan gum, alginate, dextran, acacia gum, karaya gum, locust bean gum, tragacanth, and mixtures thereof, and the like; disintegrants, such as, sodium starch glycolate, crosscarmellose, crosspovidone, and the like; high intensity sweeteners, such as, acesulfame-potasium, sucralose, saccharine, and the like; glidants, such as colloidal silicon dioxide and the like; and wetting agents, such as glyceryl mono-stearate, polysorbate, lecithin, and the like; coloring; and flavoring agents.

Examples of the lubricants include magnesium stearate, calcium stearate, stearic acid, talc, and hardened vegetable oils. The preferred lubricant is magnesium stearate. Preferably, the lubricant is present in a lactase tablet composition in an amount from about 0.1 % to about 6% and most preferably from about 0.25% to about 4% by weight.

The quantity of lactase enzyme administered in a single oral dosage can vary within wide limits. Such quantity will depend on factors that include the potency of the lactase enzyme used in the composition, the magnitude of the lactase deficiency or lactose intolerance in the particular individual requiring the dietary supplement of lactase and the dietary habits of the individual. As a general matter, the affected individual will become accustomed to estimating a required dose based on the particular facts and experience with a particular brand or source of solid dosage form.

Ideally, the lactase tablets herein should contain sufficient lactase to satisfy the dosage requirement of most individuals requiring the dietary supplement of lactase in most situations. Alternatively, the lactase tablets herein should contain a fraction of such a dose so that the gamut of affected individuals can closely match their dosage requirements with the administration of one, two or three tablets. In the first case, a tablet herein might contain 9000 FCC lactase units/tablet and, in the second case, 3000 FCC lactase units/tablet.

The solid lactase composition of the present invention can be any solid form, including, but not limited to, powder, tablet, caplet, dragee, capsule, pill, pellet, troche, lozenge, and chewy confection.

### EXAMPLES

The following examples are intended to illustrate the invention herein and are in no way intended to be limiting:

### Example 1 Comparative Formula 1:

Ingredients were weighed in the amounts listed according to Table 1 below. Spray-dried lactase concentrate (lactase enzyme 80 wt% and trisodium citrate (1) 20 wt%, obtained from Amano Pharmaceutical Co., Ltd., Naka-ku, Nagoya, Japan), trisodium citrate (2), and crystalline glucose anhydrate were combined in a plastic bottle, and mixed by shaking for 3 minutes. Microcrystalline cellulose, magnesium stearate, and colloidal silicon dioxide were added, and mixed by shaking for an additional 3 minutes. The resulting blend was compressed on a single station tablet press (Stokes Model A) using 7/16 inch round concave tooling to a weight of 210 mg, average hardness of 4.6 kp, and average thickness of 0.121 inches.

### Example 2 Inventive Formula 1:

Ingredients were weighed in the amounts listed according to Table 1 below. Spray-dried lactase concentrate and microcrystalline cellulose were combined in a plastic bottle, and mixed by shaking for 3 minutes. Additional microcrystalline cellulose, magnesium stearate, and colloidal silicon dioxide were added, and mixed by shaking for an additional 3 minutes. The resulting blend was compressed on a single station tablet press (Stokes Model A) using 7/16 inch round concave tooling to a weight of 210 mg, average hardness of 3.9 kp, and average thickness of 0.121 inches.

### Example 3

Ingredients were weighed in the amounts listed according to Table 1 below. Spray-dried lactase concentrate and dicalcium phosphate were combined in a plastic bottle, and mixed by shaking for 3 minutes. Additional microcrystalline cellulose, magnesium stearate, and colloidal silicon dioxide were added, and mixed by shaking for an additional 3 minutes. The resulting blend was compressed on a single station tablet press (Stokes Model A) using 7/16 inch round concave tooling to a weight of 210 mg, average hardness of 3.3 kp, and average thickness of 0.121 inches.

### Example 4

Ingredients were weighed in the amounts listed in the table below. Spray-dried lactase concentrate and cornstarch were combined in a plastic bottle, and mixed by shaking for 3 minutes. Additional microcrystalline cellulose, magnesium stearate, and colloidal silicon dioxide were added, and mixed by shaking for an additional 3 minutes. The resulting blend was compressed on a single station tablet press (Stokes Model A) using 7/16 inch round concave tooling to a weight of 210 mg, average hardness of 5.7 kp, and average thickness of 0,123 inches.

**Table 1**

| Compositions 1 through 4 (amounts are %w/w) | | | | |
|---|---|---|---|---|
| **Ingredient** | **Example 1** | **Example 2** | **Example 3** | **Example 4** |
| Lactase Concentrate | 28.48 | 28.48 | 28.48 | 28.48 |
| Trisodium Citrate (1) | 7.12 | 7.12 | 7.12 | 7.12 |
| Crystalline Glucose Anhydrate | 11.1 | 0 | 0 | 0 |
| Trisodium Citrate (2) | 2.6 | 0 | 0 | 0 |
| Microcrystalline Cellulose | 49.9 | 63.6 | 49.9 | 49.9 |
| Dicalcium Phosphate | 0 | 0 | 13.7 | 0 |
| Starch, NF | 0 | 0 | 0 | 13.7 |
| Magnesium Stearate | 0.5 | 0.5 | 0.5 | 0.5 |
| Colloidal Silicon Dioxide | 0.3 | 0.3 | 0.3 | 0.3 |

### Example 5 Comparative Formula 2:

One bottle of Dairy Ease chewable lactase tablets (100s) lot #00C277A, expiration date: 04/2002, was purchased at retail. Ingredients: mannitol, microcrystalline cellulose, magnesium stearate, sucrose, colloidal silicone dioxide, flavor and lactase enzyme NLT 9000 FCC units. Tablet weights ranged from 935 to 966 mg (average 949 mg).

### Example 6 Color Testing of Tablets from Examples 1 through 5

Tablets from examples 1 through 5 were stored for 7 days in open dishes at the following "stress" conditions: 25°C and 60% relative humidity, 25°C and 75% relative humidity, 25°C and 90% relative humidity, and 40°C and 75% relative humidity. After 7 days, tablets stored at cach condition were tested with the Milton-Roy Colormate Instrument (Model #347902, Milton Roy Company, Rochester NY) to determine the ASTM Yellowness Index (D1925). Results are reported below as the average of 6 tablets tested. The yellowness index of the room temperature and 60% relative humidity sample was used as a reference point to determine the percent difference for the tablets stored at the higher humidity conditions. Results indicate that under these conditions the tablets of the invention (examples 2, 3 and 4) exhibit substantially less yellowing than either of the comparator tablets (examples 1 and 5).

**Table 2:**

| ASTM Yellowness Index D1925 | | | | | |
|---|---|---|---|---|---|
| Sample | Formula 1 | Formula 2 | Formula 3 | Formula 4 | Formula 5 |
| 25°C/60%RH | 9.51 | 9.26 | 7.90 | 9.00 | 4.51 |
| 25°C/75%RH | 9.57 | 8.72 | 7.90 | 8.40 | 4.62 |
| 25°C/90%RH | 18.29 | 8.84 | 8.17 | 9.01 | 4.83 |
| 40°C/75%RH | 41.41 | 11.17 | 11.27 | 10.90 | 7.32 |
| nt = not tested | | | | | |

**Table 3:**

| Percent Change in ASTM Yellowness Index compared to 25C/60%RH sample | | | | | |
|---|---|---|---|---|---|
| Sample | Formula 1 | Formula 2 | Formula 3 | Formula 4 | Formula 5 |
| 25°C/60%RH | 0.00 | 0.00 | 0.00 | 0.00 | 0.00 |
| 25°C/75%RH | 0.63 | -5.83 | 0.00 | -6.67 | 2.44 |
| 25°C/90%RH | 92.32 | -4.54 | 3.42 | 0.11 | 7.10 |
| 40°C/75%RH | 335.44 | 20.63 | 42.66 | 21.11 | 62.31 |
| nt = not tested | | | | | |

## Claims

1. A composition for hydrolyzing lactose in a human in need thereof by oral administration comprising
a) a lactose hydrolyzing around of β-galactosidase and
b) an excipient, and
c) less than about 10 wt% of a reducing sugar,
wherein the composition undergoes a change in yellowness of about 45% or less after seven days at 40°C/75% RH as determined by ASTM D1925.

2. The composition of claim 1, wherein the change in yellowness is about 20% to about 45% after seven days at 40°C/75% RH as determined by ASTM D1925.

3. The composition of claim 1 or claim 2, wherein the reducing sugar is present in an amount of less than about 5 w%.

4. The composition of claim 3, wherein the composition is substantially free of a reducing sugar.

5. The composition of any one of claims 1 to 4 wherein the excipient is a hydrophilic non-water soluble excipient, a hydrophobic excipient or a mixture thereof.

6. The composition of any one of claims 1 to 5 wherein the excipient is hydrophobic and is talc, dicalcium phosphate, tricalcium phosphate, calcium carbonate or a mixture thereof..

7. The composition of any one of claims 1 to 5 wherein the excipient is hydrophilic and non-water soluble and is microcrystalline cellulose, cornstarch or a mixture thereof.

8. The composition of any one of claims 1 to 7 wherein the excipient is present in an amount from 10 wt% to 75 wt%, preferably from 45 wt% to 65 wt%, more preferably from 15 wt% to 30 %.

9. The composition of any one of claims 1 to 8 wherein β-galactosidase is present in an amount of about 23 wt%.

10. The composition of any one of claims 1 to 9, for use in the treatment of lactose malabsorbtion by oral administration.
